# EUROPÄISCHE PATENTSCHRIFT

(11) **EP 0 546 418 B1**
(45) Veröffentlichungstag und Bekanntmachung des Hinweises auf die Patenterteilung: **17.10.2001**
(21) Anmeldenummer: 92120435.0
(22) Anmeldetag: 30.11.1992
(51) Int. Cl.: C07D 213/61

(54) **Verfahren zur Herstellung von 5-substituierten 2-Chlorpyridinen**
Process for the preparation of 5-substituted 2-chloropyridines
Procédé pour la préparation de chloro-2 pyridines substituées en position 5

(30) Priorität: 13.12.1991 DE 4141188; 22.05.1992 DE 4217021
(43) Veröffentlichungstag der Anmeldung: 16.06.1993
(73) Patentinhaber: BAYER AG, 51368 Leverkusen (DE)
(72) Erfinder: Lantzsch, Reinhard, Dr., W-5600 Wuppertal 1 (DE)

(56) Entgegenhaltungen:
- EP-A- 0 108 483
- EP-A- 0 439 745
- JOURNAL OF THE CHEMICAL SOCIETY, PERKIN TRANSACTIONS 1 Nr. 6, 1984, LETCHWORTH GB Seiten 1173 - 1182 O. METH-COHN ET AL. 'A versatile new synthesis of quinolines and related fused pyridines. Part 12. A general synthesis of 2-chloropyridines and 2-pyridones.'

## Beschreibung

Die Erfindung betrifft ein neues Verfahren zur Herstellung von 5-substituierten 2-Chlor-pyridinen.

Es ist bekannt, daß man 5-substituierte 2-Chlor-pyridine, wie z.B. 5-Methyl-2-chlor-pyridin, erhält, wenn man entsprechende Pyridin-1-oxide, wie z.B. 3-Methyl-pyridin-1-oxid, mit Phosphorylchlorid (vgl. EP-A 324174) oder mit anderen Säurechloriden (vgl. EP-A 438691 und EP-A 439745 sowie dort zitierter Stand der Technik) umsetzt.

Die 5-substituierten 2-Chlor-pyridine fallen hierbei jedoch immer mehr oder weniger stark verunreinigt an.

Weiter sind verschiedene mehrstufige Synthesewege zu 5-Methyl-2-chlor-pyridin bekannt geworden (vgl. EP-A 108483, EP-A 121320 und EP-A 162464).

Diese Synthesen sind jedoch relativ aufwendig und verlustreich.

Ferner ist bekannt, daß aus bestimmten Enamiden mit Phosphorylchlorid/Dimethylformamid Gemische aus Pyridonen und Pyridinen gebildet werden (vgl. J. Chem. Soc. Perkin Trans. I 1984, 1173-1182). Hierbei werden jedoch vorwiegend Pyridone erhalten, während Pyridine nur als Nebenprodukte anfallen.

Es wurde nun gefunden, daß man 5-substituierte 2-Chlor-pyridine der allgemeinen Formel (I) 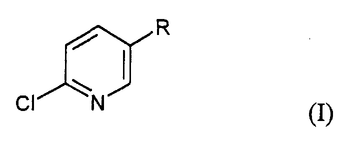 in welcher
- R: für jeweils gegebenenfalls durch Fluor und/oder Chlor substituiertes C₁-C₆-Alkyl oder Phenyl-C₁-C₂-alkyl steht,
auf einfache Weise in guten Ausbeuten erhält, wenn man Acetenamide der allgemeinen Formel (II) 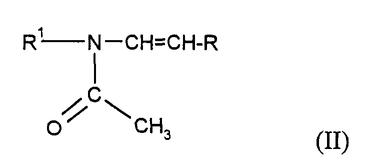 in welcher
- R: die oben angegebene Bedeutung hat und
- R¹: für jeweils gegebenenfalls durch Fluor und/oder Chlor substituiertes C₁-C₄-Alkyl oder Benzyl steht,
mit einem Chlorierungsmittel der Reihe Phosphorylchlorid(Phosphoroxychlorid/-POCl₃), Phosphor(V)-chlorid, Phosgen, Oxalylchlorid, Thionylchlorid, organische Polyhalogenverbindungen wie Perchlorbutansäurechlorid und Dichlorbenzodioxiol in Gegenwart von N,N-disubstituierten Formamiden der Reihe Dimethylformamid, Dibutylformamid oder Phenylmethylformamid und gegebenenfalls in Gegenwart eines Verdünnungsmittels bei Temperaturen in der ersten Umsetzungsphase von 0°C bis 20°C und dann bei 80°C bis 120°C umsetzt, wobei man pro Mol Acetenamid der Formel (II) 1 bis 10 Mol Chlorierungsmittel und 2 bis 20 Mol N,N-disubstituiertes Formamid einsetzt.

In Anbetracht des oben zitierten Standes der Technik (vgl. J. Chem. Soc. Perkin Trans. I 1984, 1173-1182) ist es als ausgesprochen überraschend anzusehen, daß nach dem erfindungsgemäßen Verfahren die 5-substituierten 2-Chlor-pyridine der Formel (I) als Hauptprodukte in guten Ausbeuten erhalten werden.

Im Gegensatz zu den oben erwähnten bekannten Verfahren kann die Herstellung der Ausgangsstoffe der Formel (II) ausgehend von handelsüblichen, großtechnisch zugänglichen Vorprodukten erfolgen, wobei eine Isolierung der Zwischenstufen als Reinsubstanzen nicht erforderlich ist. Das erfindungsgemäße Verfahren stellt somit eine wertvolle Bereicherung des Standes der Technik dar.

Der Reaktionsablauf beim erfindungsgemäßen Verfahren kann beispielsweise durch das folgende Formelschema skizziert werden:

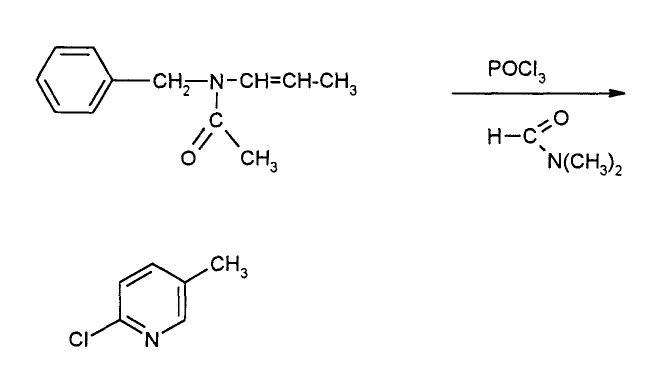

Das erfindungsgemäße Verfahren betrifft vorzugsweise die Herstellung von Verbindungen der Formel (I) in welcher
- R: für Methyl, Ethyl oder Benzyl steht.

Die als Ausgangsstoffe zu verwendenden Acetenamide sind durch die Formel (II) allgemein definiert. In Formel (II) stehen vorzugsweise
- R: für Methyl, Ethyl oder Benzyl und
- R¹: für Benzyl.

Die Ausgangsstoffe der Formel (II) sind bekannt und/oder können nach an sich bekannten Verfahren hergestellt werden (vgl. J. Chem. Soc. Perkin Trans. I 1984, 1173 - 1182).

Man erhält die Acetenamide der Formel (II) beispielsweise, wenn man Imine der allgemeinen Formel (III)

R¹-N=CH-CH₂-R (III)

in welcher
- R und R¹: die oben angegebene Bedeutung haben
mit Acetanhydrid oder Acetylchlorid, gegebenenfalls in Gegenwart eines Säureakzeptors, wie z.B. Triethylamin, und gegebenenfalls in Gegenwart eines Verdünnungsmittels, wie z.B. Toluol, bei Temperaturen zwischen 0°C und 50°C umsetzt und nach üblichen Methoden aufarbeitet (vgl. die Herstellungsbeispiele).

Die Imine der Formel (III) sind bekannt und/oder können nach an sich bekannten Verfahren hergestellt werden (vgl. Chem. Abstracts 88:49759; Herstellungsbeispiele).

Chlorierungsmittel, die beim erfindungsgemäßen Verfahren eingesetzt werden können, sind Verbindungen, welche mit Formamiden oder anderen N,N-disubstituierten Formamiden das sogenannte Vilsmeier-Reagenz (N,N-disubstituiertes Chlormethylimmoniumchlorid) (IV) 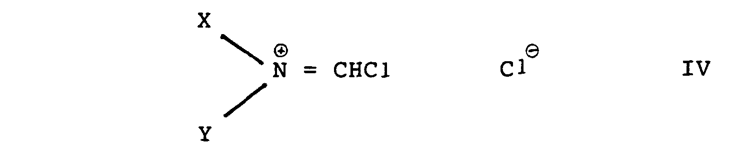 wobei
X und Y für C₁₋₄-Alkyl oder Phenyl stehen bilden.

Als Formamide kommen infrage Dimethylformamid, Dibutylformamid, Phenylmethylformamid. Bevorzugt ist N,N-Dimethylformamid.

Hierzu gehören insbesondere Säurechloride, wie Phosphorylchlorid (Phosphoroxychlorid/POCl₃), Phosphor(V)-chlorid, Phosgen, Oxalylchlorid und Thionylchlorid, aber auch organische Polyhalogenverbindungen, wie Perchlorbutansäurechlorid und Dichlorbenzodioxol. Besonders bevorzugt werden Phosphorylchlorid, Oxalylchlorid und Phosgen.

Das erfindungsgemäße Verfahren zur Herstellung der 5-substituierten 2-Chlor-pyridine der Formel (I) wird gegebenenfalls in Gegenwart eines Verdünnungsmittels durchgeführt. Es kommen praktisch alle für die Reaktion inerten organischen Lösungsmittel in Betracht. Hierzu gehören vorzugsweise aliphatische und aromatische, gegebenenfalls halogenierte Kohlenwasserstoffe wie Hexan, Heptan, Cyclohexan, Methylcyclohexan, Benzol, Toluol, Xylol, Chlorbenzol, o-Dichlorbenzol, Chloroform und Tetrachlormethan, sowie Ether wie Methyl-tert-butylether und 1,2-Dimethoxyethan.

Vorzugsweise wird jedoch auf die Verwendung eines inerten Verdünnungsmittels verzichtet, d.h. die Umsetzung wird in überschüssigem N,N-disubstituiertem Formamid als Verdünnungsmittel durchgeführt.

Die Reaktionstemperaturen können bei dem erfindungsgemäßen Verfahren in einem größeren Bereich variiert werden. Man arbeitet bei Temperaturen in der ersten Umsetzungsphase bei 0°C bis 20°C, dann bei 80°C bis 120°C.

Das erfindungsgemäße Verfahren wird im allgemeinen unter Normaldruck durchgeführt. Es ist jedoch auch möglich, unter erhöhtem oder vermindertem Druck zwischen 0,1 bar und 10 bar zu arbeiten.

Zur Durchführung des erfindungsgemäßen Verfahrens setzt man auf 1 Mol Acetenamid der Formel (II) im allgemeinen zwischen 1 und 10 Mol, vorzugsweise zwischen 1,5 und 5 Mol, insbesondere zwischen 2,0 und 3,0 mol Chlorierungsmittel und zwischen 2 und 20 Mol Dimethylformamid ein.

In einer bevorzugten Ausführungsform des erfindungsgemäßen Verfahrens werden zunächst das Chlorierungsmittel und das Dimethylformamid zur Umsetzung gebracht, vorzugsweise indem das Dimethylformamid vorgelegt wird und das Chlorierungsmittel unter leichtem Kühlen langsam dazu gegeben wird. In diese Mischung wird dann das Acetenamid der Formel (II) langsam eindosiert und das Reaktionsgemisch wird bei erhöhter Temperatur bis zum Ende der Umsetzung gerührt.

Die Aufarbeitung kann auf übliche Weise durchgeführt werden. Beispielsweise wird mit Eiswasser verdünnt, mit einem mit Wasser praktisch nicht mischbaren organischen Lösungsmittel, wie z.B. Methylenchlorid, extrahiert und die Extraktionslösung eingeengt. Das Produkt der Formel (I) kann aus dem verbleibenden Rückstand durch Vakuumdestillation rein erhalten werden.

Die nach dem erfindungsgemäßen Verfahren herzustellenden 5-substituierten 2-Chlor-pyridine können als Zwischenprodukte für Insektizide verwendet werden (vgl. EP-A 163855).

### Herstellungsbeispiele:

### Beispiel 1

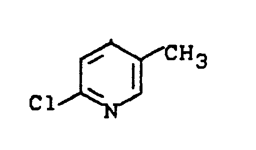

214 g (1,39 Mol) Phosphoroxychlorid werden bei einer Innentemperatur von 5°C bis 10°C innerhalb von 50 Minuten zu 565 ml (7,33 Mol) Dimethylformamid tropfenweise gegeben. Dann werden bei der gleichen Temperatur 113,7 g N-Benzyl-N-(1-propenyl)-acetamid (Rohprodukt gemäß Beispiel (II-1)) zugetropft. Das Reaktionsgemisch wird 16 Stunden bei 100°C gerührt und nach dem Abkühlen auf ca. 1 Liter Eiswasser gegossen. Man extrahiert viermal mit Methylenchlorid und engt die vereinigten Extraktionslõsungen ein.

Man erhält 112 g einer dunklen Flüssigkeit, die nach gaschromatographischer Analyse zu 60,4 Gew.-% aus Benzylchlorid und zu 35,7 Gew.-% aus 2-Chlor-5-methylpyridin besteht.

Dies entspricht einer Ausbeute von 67,5% der Theorie über die beiden letzten Stufen (Beispiele (II-1) und 1).

Nach zweifacher Destillation - zunächst im Wasserstrahlvakuum über einen Siedebereich zwischen 40°C und 82°C, dann über eine Spaltrohrkolonne (Siedepunkt 118°C bei 60 mbar) erhält man reines 2-Chlor-5-methyl-pyridin (Gehalt: 97,3%).

### Beispiel 2

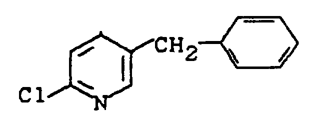

196 g (1,23 Mol) Phosphoroxychlorid werden bei 5°C bis 10°C innerhalb von 45 Minuten zu 112 g (1,53 Mol) Dimethylformamid tropfenweise gegeben. Dann werden bei der gleichen Temperatur 137 g N-Benzyl-N-(3-phenyl-1-propenyl)-acetamid (Rohprodukt gemäß Beispiel (II-2)), innerhalb von 30 Minuten zugetropft. Das Reaktionsgemisch wird 12 Stunden bei 100°C gerührt und nach dem Abkühlen auf ca. 0,5 Liter Eiswasser gegossen. Man extrahiert viermal mit Methylenchlorid und engt die vereinigten Extraktionslösungen ein.

Man erhält 121 g einer dunklen Flüssigkeit, die nach gaschromatographischer Analyse 56,2 Gew.-% 2-Chlor-5-benzyl-pyridin und 29,7 Gew.-% Benzylchlorid enthält. Durch Vakuumdestillation erhält man 32 g farbloses Öl vom Siedepunkt 132°C-134°C/1 mbar (Gehalt: 95,6% 2-Chlor-5-benzyl-pyridin), entsprechend einer Ausbeute von 34 % der Theorie über die beiden letzten Stufen (Beispiel (II-2) und 2), hierbei wird von 97,2 g (0,465 Mol) der Verbindung III-2 ausgegangen.

Analog zu den Beispielen 1 und 2 sowie entsprechend der allgemeinen Beschreibung des erfindungsgemäßen Verfahrens können beispielsweise auch die in der nachstehenden Tabelle 1 aufgeführten Verbindungen der Formel (I) hergestellt werden.

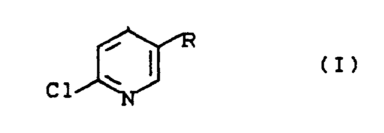

**Tabelle 1**

| Beispiele für die erfindungsgemäß herzustellenden Verbindungen der Formel (I) | | | |
|---|---|---|---|
| Bsp.-Nr. | R | Siedepunkt | Ausbeute der 2 letzten Stufen |
| 3 | C₂H₅ | 95°C-100°C (bei 12 mbar) | 49% |
| 4 | C₅H₁₁ | | 12% |

### Beispiel 5

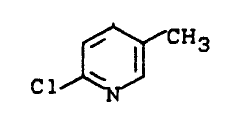

13,3 g (0,105 Mol) Oxalylchlorid werden bei einer Innentemperatur von 0°C bis +5°C innerhalb von 40 Minuten zu 40,9 g (0,56 Mol) Dimethylformamid tropfenweise gegeben. Dann werden bei der gleichen Temperatur 6,6 g N-Benzyl-N-(1-propenyl)-acetamid (Rohprodukt gemäß Beispiel (II-1)) zugetropft. Das Reaktionsgemisch wird dann 12 Stunden bei 100°C gerührt und nach dem Abkühlen auf 140 g Eiswasser gegossen. Man extrahiert viermal mit Methylenchlorid und engt die vereinigten Extraktionslösungen im Wasserstrahlvakuum ein.

Man erhält 10,9 g eines braungelben flüssigen Rückstandes, der nach gaschromatographischer Analyse 28,2% 2-Chlor-5-methyl-pyridin enthält. Bezogen auf die Reinheit des Ausgangsstoffes (Gehalt: 73% an (II)) ergibt sich damit eine Ausbeute von 93% der Theorie,

### Beispiel 6

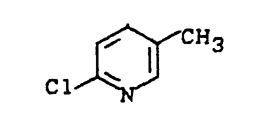

10,4 g (0,105 Mol) Phosgen und 40,9 g (0,56 Mol) Dimethylformamid werden zunächst bei 0°C bis +5°C umgesetzt. Dann werden bei der gleichen Temperatur 6,6 g N-Benzyl-N-(1-propenyl)-acetamid (Rohprodukt gemäß Beispiel (II-1)) zugetropft. Das Reaktionsgemisch wird dann 12 Stunden bei 100°C gerührt und anschließend wie bei Beispiel 5 beschrieben aufgearbeitet.

Man erhält so 2-Chlor-5-methyl-pyridin in einer Ausbeute von 92% der Theorie.

### Ausgangsstoffe der Formel (II)

### Beispiel (II-1)

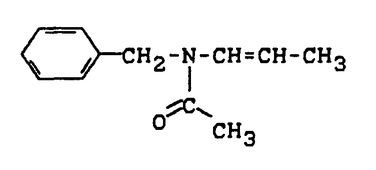

68,4 g (0,465 Mol) N-Propyliden-benzylamin (vgl. Beispiel (III-1)) werden in 250 ml Toluol gelöst. Diese Lösung wird auf ca. 5°C abgekühlt und bei dieser Temperatur werden nacheinander 47,1 g (0,465 Mol) Triethylamin und 47,4 g (0,465 Mol) Acetanhydrid, jeweils innerhalb von 30 Minuten, zugetropft. Anschließend wird das Reaktionsgemisch noch ca. 3 Stunden bei 20°C gerührt. Dann werden die leichter flüchtigen Komponenten bei 50°C/ 2 mbar abdestilliert.

Man erhält 113,7 g eines gelben, öligen Rückstands, der im wesentlichen aus N-Benzyl-N-(1-propenyl)-acetamid besteht und der ohne weitere Reinigung für die Folgestufe (vgl. Beispiel 1) eingesetzt wird. Das Produkt kann durch Vakuumdestillation (Siedebereich: 120°C-130°/ 1 mbar) und anschließende Säulenchromatographie (Kieselgel; Petrolether/Essigsäureethylester, 2:1) gereinigt werden.

Analog Beispiel (II-1) können beispielsweise auch die in der nachstehenden Tabelle 2 aufgeführten Verbindungen der Formel (II) hergestellt werden.

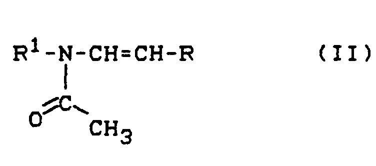

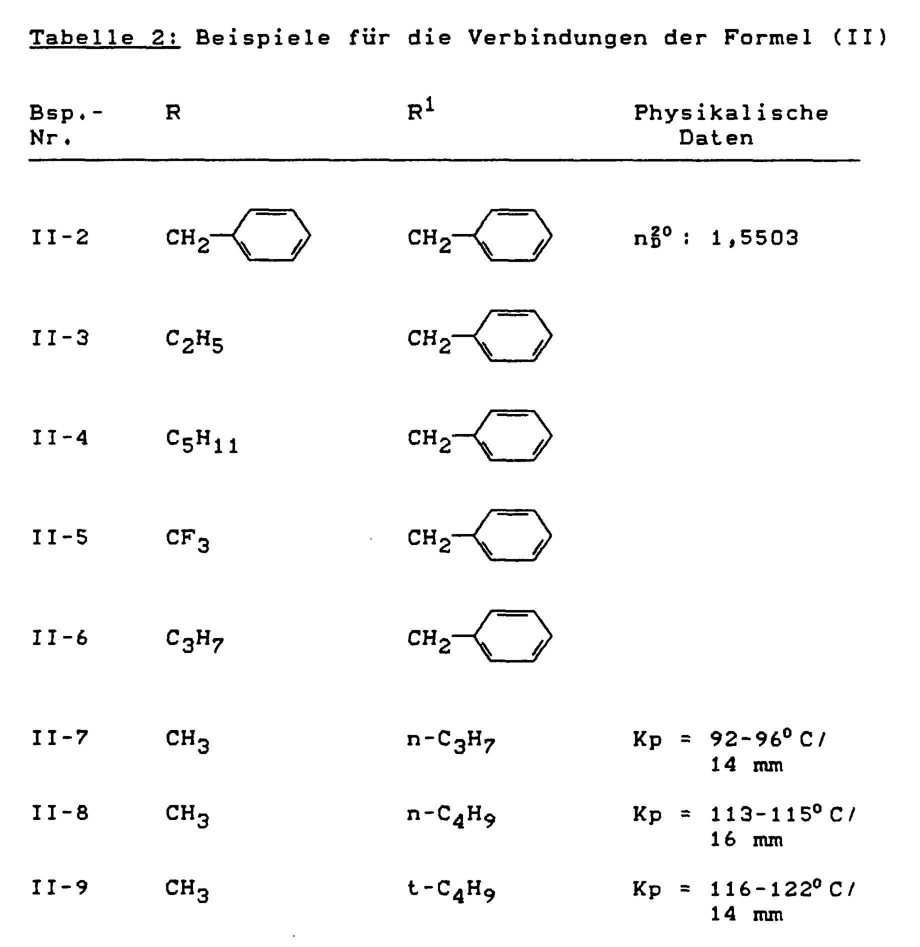

### Vorprodukte der Formel (III) :

### Beispiel III-1

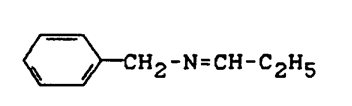

53,5 g (0,5 Mol) Benzylamin werden auf 5°C abgekühlt und unter Rühren mit 29 g (0,5 Mol) Propionaldehyd tropfenweise versetzt. Man läßt das Gemisch auf Raumtemperatur kommen und gibt 15 g Kaliumhydroxid (Plätzchen) dazu. Dann wird die untere (wäßrige) Phase abgetrennt, die organische Phase mit weiteren 5 g Kaliumhydroxid versetzt und über Nacht stehen gelassen.

Durch Abdekantieren erhält man 68,6 g (93% der Theorie) N-Propyliden-benzylamin als klare Flüssigkeit, die nicht unzersetzt destilliert werden kann und daher direkt weiter (vgl. Beispiel (II-1)) umgesetzt wird.

Analog Beispiel (III-1) können beispielsweise auch die in der nachstehenden Tabelle 3 aufgeführten Verbindungen der Formel (III) hergestellt werden.

R¹-N=CH-CH₂-R (III)

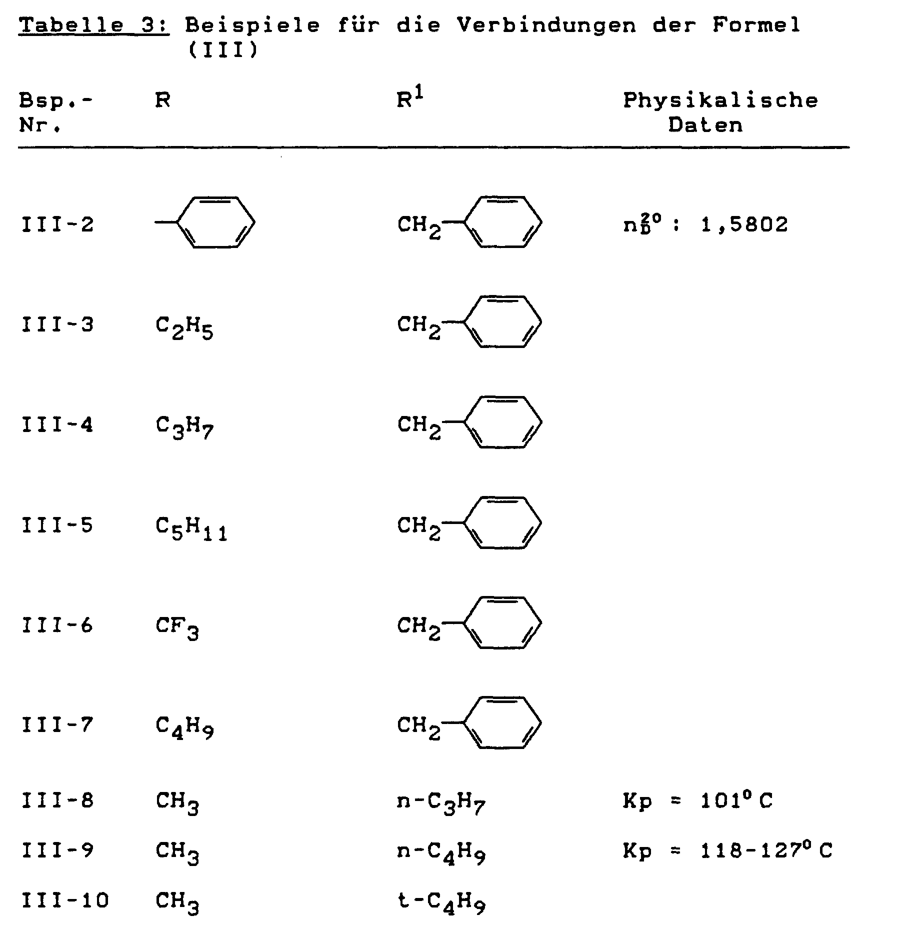

## Patentansprüche

1. Verfahren zur Herstellung von 5-substituierten 2-Chlorpyridinen der allgemeinen Formel (I) 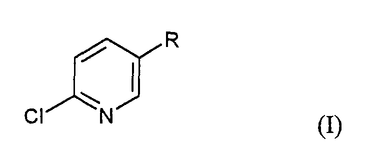 in welcher
R für jeweils gegebenenfalls durch Fluor und/oder Chlor substituiertes C₁-C₆-Alkyl oder Phenyl-C₁-C₂-alkyl steht,
**dadurch gekennzeichnet, daß** man Acetenamide der allgemeinen Formel (II) 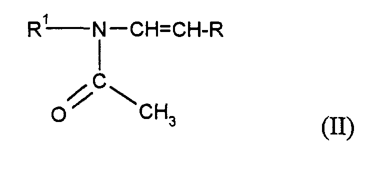 in welcher
R die oben angegebene Bedeutung hat und
R¹ für jeweils gegebenenfalls durch Fluor und/Chlor substituiertes C₁-C₄-Alkyl oder Benzyl steht,
mit einem Chlorierungsmittel der Reihe Phosphorylchlorid, Phosphor(V)-chlorid, Phosgen, Oxalylchlorid, Thionylchlorid, Perchlorbutansäurechlorid oder Dichlorbenzodioxiol in Gegenwart eines N,N-disubstituierten Formamids der Reihe Dimethylformamid, Dibutylformamid oder Phenylmethylformamid, gegebenenfalls in Gegenwart eines Verdünnungsmittels bei Temperaturen in der ersten Umsetzungsphase von 0°C bis 20°C und dann bei 80°C bis 120°C umsetzt, wobei man pro Mol Acetenamid der Formel (II) 1 bis 10 Mol Chlorierungsmittel und 2 bis 20 Mol N,N-disubstituiertes Formamid einsetzt.

2. Verfahren gemäß Anspruch 1, **dadurch gekennzeichnet, daß** man die Umsetzung ohne zusätzliches Verdünnungsmittel in überschüssigem N-N-disubstituiertem Formamid durchführt.

3. Verfahren gemäß Anspruch 2, **dadurch gekennzeichnet, daß** man Dimethylformamid verwendet.

4. Verfahren gemäß Anspruch 1, **dadurch gekennzeichnet, daß** man Verbindungen der Formel (I) herstellt, in welcher R für Methyl, Ethyl oder Benzyl steht.

5. Verfahren gemäß Anspruch 1, **dadurch gekennzeichnet, daß** man pro Mol Acetenamid 1,5 bis 5 Mol Phosphorylchlorid, Phosgen oder Oxalylchlorid und 2 bis 15 Mol Dimethylformamid einsetzt.

6. Verfahren gemäß Anspruch 1 und 5, **dadurch gekennzeichnet, daß** man das Phosphorylchlorid, oder Oxalylchlorid oder Phosgen unter leichtem Kühlen langsam dazugibt, und in diese Mischung das Acetenamid der Formel (II) langsam eindosiert und das Reaktionsgemisch bei erhöhter Temperatur bis zum Ende der Umsetzung rührt.

## Claims

1. Process for the preparation of 5-substituted 2-chloropyridines of the general formula (I) 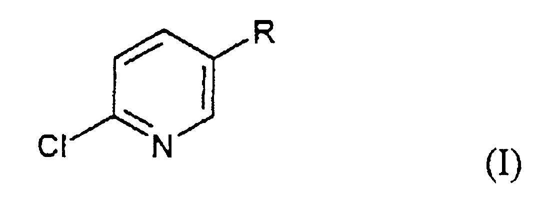 in which
R represents C₁-C₆-alkyl or phenyl-C₁-C₂-alkyl, each of which is optionally substituted by fluorine and/or chlorine,
**characterised in that** acetenamides of the general formula (II) 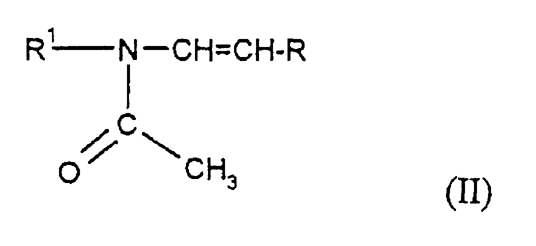 in which
R has the abovementioned meaning, and
R¹ represents C₁-C₄-alkyl or benzyl, each of which is optionally substituted by fluorine and/or chlorine,
are reacted with a chlorinating agent from the group consisting of phosphoryl chloride, phosphorus(v) chloride, phosgene, oxalyl chloride, thionyl chloride, perchlorobutanoyl chloride and dichlorobenzodioxole in the presence of an N, N-disubstituted formamide from the group consisting of dimethylformamide, dibutylformamide and phenylmethylformamide and optionally in the presence of a diluent at temperatures in the first phase of the reaction of 0°C to 20°C and then at 80°C to 120°C, 1 to 10 moles of chlorinating agent and 2 to 20 moles of N, N-disubstituted formamide being used per mole of acetenamide of the formula (II).

2. Process according to Claim 1, **characterised in that** the reaction is performed in excess N, N-disubstituted formamide without an additional diluent.

3. Process according to Claim 2, **characterised in that** dimethylformamide is used.

4. Process according to Claim 1, **characterised in that** compounds of the formula (I) are prepared in which R represents methyl, ethyl or benzyl.

5. Process according to Claim 1, **characterised in that** 1.5 to 5 moles of phosphoryl chloride, phosgene or oxayl chloride and 2 to 15 moles of dimethylformamide are used per mole of acetenamide.

6. Process according to Claims 1 to 5, **characterised in that** the phosphoryl chloride, or oxalyl chloride or phosgene, is added slowly with slight cooling, the acetenamide of the formula (II) is metered slowly into this mixture and the reaction mixture is stirred at elevated temperature until the end of the reaction.

## Revendications

1. Procédé de production de 2-chloropyridines substituées en position 5, de formule générale (I) 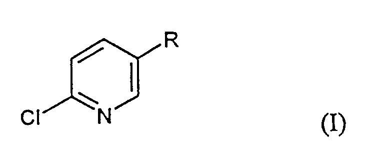 dans laquelle
R représente un groupe alkyle en C₁ à C₆ ou un groupe phényl- (alkyle en C₁ ou C₂) dont chacun est éventuellement substitué par du fluor et/ou du chlore,
**caractérisé en ce qu'**on fait réagir des acéténamides de formule générale (II) 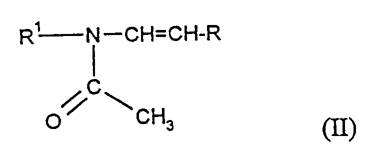 dans laquelle
R a la définition indiquée ci-dessus et
R¹ représente un groupe alkyle en C₁ à C₄ ou un groupe benzyle dont chacun est éventuellement substitué par du fluor et/ou du chlore,
avec un agent de chloration de la série chlorure de phosphoryle, chlorure de phosphore(V), phosgène, chlorure d'oxalyle, chlorure de thionyle, chlorure d'acide perchlorobutanoïque ou dichlorobenzodioxiole en présence d'un formamide N,N-disubstitué de la série diméthylformamide, dibutylformamide ou phénylméthylformamide, le cas échéant en présence d'un diluant à des températures de 0°C à 20°C dans la première phase de réaction, puis à une température de 80°C à 120°C, en utilisant par mole d'acéténamide de formule (II) 1 à 10 moles d'agent de chloration et 2 à 20 moles de formamide N,N-disubstitué.

2. Procédé suivant la revendication 1, **caractérisé en ce qu'**on conduit la réaction dans du formamide N,N-disubstitué en excès, sans addition de diluant.

3. Procédé suivant la revendication 2, **caractérisé en ce qu'**on utilise du diméthylformamide.

4. Procédé suivant la revendication 1, **caractérisé en ce qu'**on produit des composés de formule (I) dans laquelle R est un groupe méthyle, éthyle ou benzyle.

5. Procédé suivant la revendication 1, **caractérisé en ce qu'**on utilise par mole d'acéténamide 1,5 à 5 moles de chlorure de phosphoryle, de phosgène ou de chlorure d'oxalyle et 2 à 15 moles de diméthylformamide.

6. Procédé suivant les revendications 1 et 5, **caractérisé en ce qu'**on ajoute lentement le chlorure de phosphoryle ou le chlorure d'oxalyle ou le phosgène en refroidissant légèrement et on verse lentement l'acéténamide de formule (II) en quantité dosée dans ce mélange et on agite le mélange réactionnel à température élevée jusqu'à la fin de la réaction.
